# EUROPEAN PATENT SPECIFICATION

(11) **EP 2 640 822 B1**
(45) Date of publication and mention of the grant of the patent: **14.08.2019**
(21) Application number: 11799835.1
(22) Date of filing: 15.11.2011
(51) Int. Cl.: C12G 1/02, C12M 1/04

(54) **IMPROVED FERMENTATION METHOD AND FERMENTER**
VERBESSERTES FERMENTATIONSVERFAHREN UND GÄRBEHÄLTER
PROCÉDÉ DE FERMENTATION ET FERMENTEUR AMÉLIORÉS

(30) Priority: 18.11.2010 IT VR20100222
(43) Date of publication of application: 25.09.2013
(73) Proprietor: Noform S.r.l., 30020 Meolo (VE) (IT)
(72) Inventor: CROSATO, Remo, I-30020 Meolo (VE) (IT)
(74) Representative: Citron, Massimiliano
(86) International application number: PCT/IB2011/055080
(87) International publication number: WO 2012/066468

(56) References cited:
- EP-A1- 1 314 778
- EP-A1- 1 964 914
- EP-A1- 2 009 089
- EP-A1- 2 058 385
- EP-A2- 1 028 162
- WO-A1-2006/087601
- WO-A1-2010/105675
- WO-A1-2010/105676
- DE-A1- 3 415 699

## Description

The invention relates to a method of treating a vegetable product in the form of crushed material, and a fermenter that can carry it out. We will discuss an example crushed grape material.

WO 2010 105675 describes a well-known fermenter and fermentation method. The crushed grapes are put inside a tank, in pressurized environment, ferment and create a solid floating layer, called cap, which must be crushed. To not use mechanical members the cap is softly broken by an artificial jet of gas that has been accumulated during fermentation.

The gases, especially CO₂, is collected into a second tank in communication with the first through a delivery pipe, and from there poured under pressure, through another return pipe, in the first tank under the cap.

To transfer the accumulated gas maneuvers on valves are necessary: the delivery pipe is closed, the return pipe opens and the pressure in the first tank lowers for getting a pressure differential.

Despite the excellent results in winemaking, it would be useful to simplify and/or modify the method and the fermenter by varying the phases and by limiting the components, sensors and electromechanical drives. This to find process variants still unexplored and/or to execute those already discovered by fermenters with lower manufacturing costs.

The main object of the invention is to modify the method and fermenter WO 2010 105675 to simplify it, preferably while maintaining the same processing capacity.

EP2009089 discloses a tank wherein accumulated fermentation gas overflows from a pocket obtained by composing tank modules.

Another object is to develop the method and fermenter in WO 2010 105675 to obtain new process variants and/or improve performance thereof.

The objects are achieved by a method according to claim 1, which exploits a phase of setting a volume, adapted to accumulate gaseous products of fermentation generated by the pressed material, into constant and free communication (i.e. with a transmission of fluid always allowed) with the liquid mass of the pressed material at a point under the cap.

Not only some means (e.g. valves) designed to permit or deny communication between the volume and the tank (with its expensive and complicated controls) are eliminated, but also the method is faster by skipping their opening phase.

The aforementioned inventive concept is the basis for many variants that exploit the advantageous position and/or structure of the volume adapted to accumulate the gaseous products.

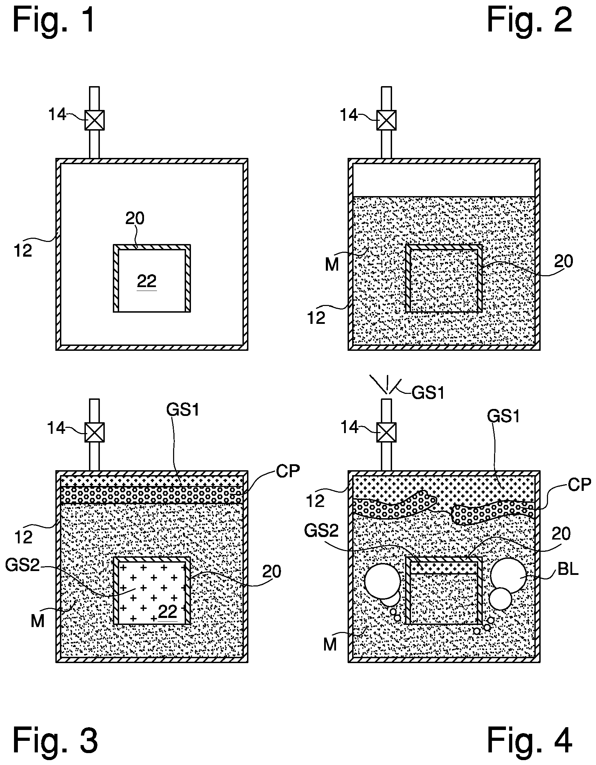

See fig. 1. For example a gas accumulation volume, represented by an open-bottom tank 20, can be placed inside a fermentation tank 12. The tank 12 allows the fermentation under pressure, i.e. it is closed or tightly closable to let the gas pressure increase inside it, and can be degassed through a vent valve 14. The tank 20 has an opening (the bottom) facing the bottom of the tank 12, so it exhibits a cavity 22 for trapping gas bubbles during their ascent.

Fig. 2 shows the tank 12 filled with must M up to a level that will cover the tank 20.

After some time (Fig. 3) above the liquid must M a floating cap CP appears having above an accumulation of gas GS1 given off from the fermenting must M. The gas bubbles rising in the must M in part get intercepted by the opening of the tank 20, whose cavity 22 is gradually filled by gas GS2 until it possibly overflows.

By opening the valve 14 (Fig. 4) the pressure in the tank 12 drops markedly, and the same drop is reflected on that acting on the gas GS2. The pressure drop results in a volume increase for the gas GS2, which comes out tumultuously from the cavity 22 and reaches the cap CP forming macro-bubbles BL that break it.

The valve 14 is closed, and the cycle can begin again.

Because the gas naturally rising in the liquid fermenting mass is directly captured inside the volume/tank 20, the distances to be covered by conveying pipes to inject the gas are zeroed. In fact these pipes disappear entirely, ditto for their possible control valves. In addition to material savings, the gas given off with bubbles BL does not enter the tank 12 through a pipe, thus through a small section, but it expands in the must M starting from the wide opening of the tank 20. Therefore, the mass of must M and cap CP involved by the bubbles BL is greater (the mixing of the must is always advantageous). And higher is the gas flow rate hitting the must M and the cap CP per unit of time, because in practice it's like having a gas injection pipe with a diameter as wide as the (very large) opening of tank 20. Another advantage is that the tank 20 discharges the gas at the center of the tank 12, fully involving the must M and the cap CP. To achieve the same result by an outer injection pipe with so big a mouth has several construction problems.

Note also the compactness of the solution, for which two (initially empty) bulky volumes are not needed but only one.

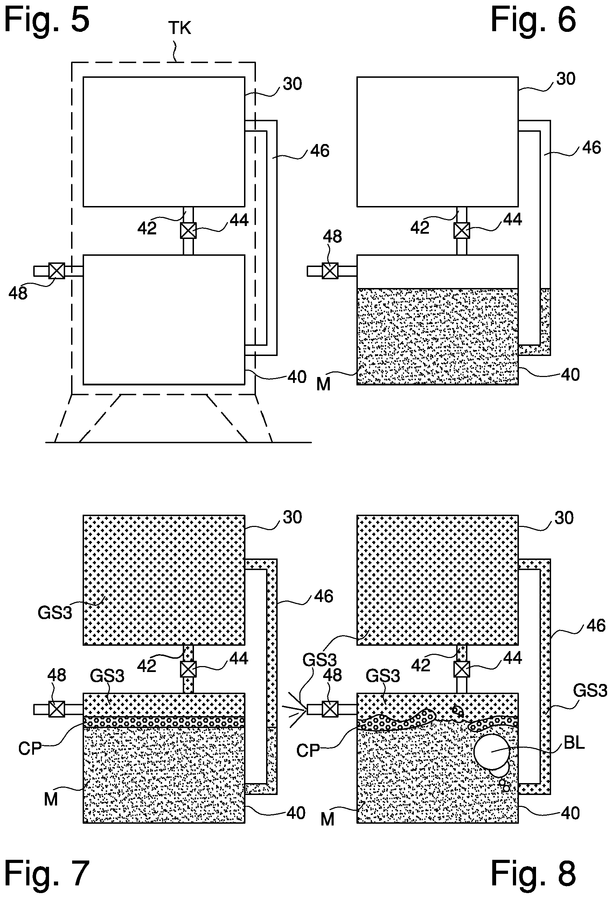

Another variant (Figs. 5 8) is connecting to a main fermentation tank 40 an external gas accumulation tank 30 via a connection 42 (e.g. a duct) interruptible by a valve 44. Another valve 48 allows to vent tank 40 to the outside or to keep it with gas under pressure inside, and a conduit 46 always open (without valves) keeps in constant communication the two tanks 30, 40 flowing into the tank 40 at a point that is in use under the cap.

After filling the tank 40 with must M, it is closed or isolated from the outside by closing the valve 48, see Fig. 6. The tank 30 is preferably arranged above the fermentation tank 40: thanks to the difference in level there is no flow of must M in the tank 30 through any of the ducts 42, 46.

The tank 30 can also be placed under the tank 40, e.g. sufficing that the two interconnecting pipes form a siphoned connection, thereby preventing the flow of the must from the top winemaking cell to the bottom gas storage cell.

Then, see Fig. 7, the valve 44 opens and the gaseous fermentation products GS3 are allowed to migrate spontaneously in the volume of the tank 30. At the same time, as the fermentation proceeds, a cap CP is formed and the pressure of gas GS3 increases.

Then the valve 44 is closed and the valve 48 is opened, so that the pressure in the tank 40 drops markedly (Fig. 8). The gas GS3 moves tumultuously from the tank 30 to tank 40, with bubbles BL breaking the cap CP and then exiting from the valve 48.

After closing the valve 48 and opening the valve 44 the cycle can begin again.

Advantageously the tanks 30, 40 can be two storage cells enclosed in, or two partitions of, a single winemaking apparatus TK, drawn dashed in Fig. 5.

Note that for this variant, and also in general, the elimination of the valves on the gas injection pipes increases the efficiency of the method and the fermenter, because the pressure drops and gas leakages (plus the costs) decrease.

The variants described here and below for the storage volume can be advantageously used alone or in combination, thereby providing a great design freedom to adapt the fermenter to any operating needs.

As general indicative values, the venting phase of the fermentation tank lasts for a time ranging from 0 to 10 seconds (depending on the type of venting/degassing means), and it is preferred to restore in it the atmospheric pressure.

The fast degassing phase also induces the release of bubbles of CO2 from the must (like when a bottle is uncorked), which adds to those leaving the accumulation volume. Together they cooperate to break the cap CP, which is sometimes so compact as to require a force of rupture and mixing so great that only the combined action of the two effervescences can achieve.

The advantages of the invention will become more clear from the following detailed description of a preferred variant, with the help of the annexed drawing in which
Figs. 1-4 show a diagram of a first fermenter according to the invention in different working phases,
Figs. 5-8 show a diagram of a second fermenter according to the invention in different working phases,
Figs. 9-13 show an internal diagram of a third fermenter according to the invention in different working phases.

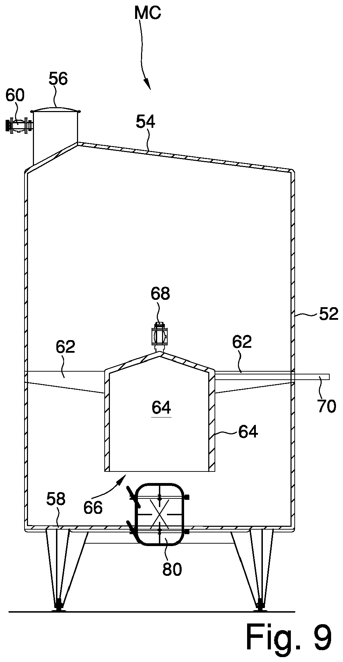

See Figures 9-13, which represent a preferred fermenter MC comprising a fermentation tank 52 having a ceiling 54 and a bottom 58. On the bottom 58 there is a manhole 80 for the discharge of marc, while on the ceiling 54 there is a vent or access manhole 56 and a valve 60 capable of venting the tank 52.

A bell-shaped tank or element 64 is suspended by arms or brackets 62 inside and at the center of the tank 52, e.g. approximately at one third of the height starting from the bottom 58. The tank 64 is bottomless, and has an opening 66 facing the bottom 58. A valve 68 is mounted on top of the tank 68 and serves to open/close a passage from inside the tank 64 in the direction of the ceiling 54. In place of the tank 64 it can also be used a cap, or in general a container or receptacle, with one open side.

The means for suspending the tank 64 can be chosen with some freedom, e.g. something like cables, lattices or legs resting on the bottom 58. Preferably the means for suspending or supporting the tank 64 allow a non-permanent connection with the inside of the tank 52 and/or with the tank 64. This is to facilitate the disassembly of the components inside the tank 52 for maintenance or cleaning.

By a conduit or pipe 70 the inner cavity 64 of tank 64 is made to communicate, e.g. selectively by valves, with the outside of the tank 52.

Proven optimal values for the tank 64: it can have an internal volume equal to 5% up to 30% of the total volume of the tank 54 (according to the types of grapes and caps to break); and a width from 30% up to 60% of the tank 54.

The position of the tank 64 has no strict constraints, it suffices - as we will explain - that it is able to trap gas rising in the must.

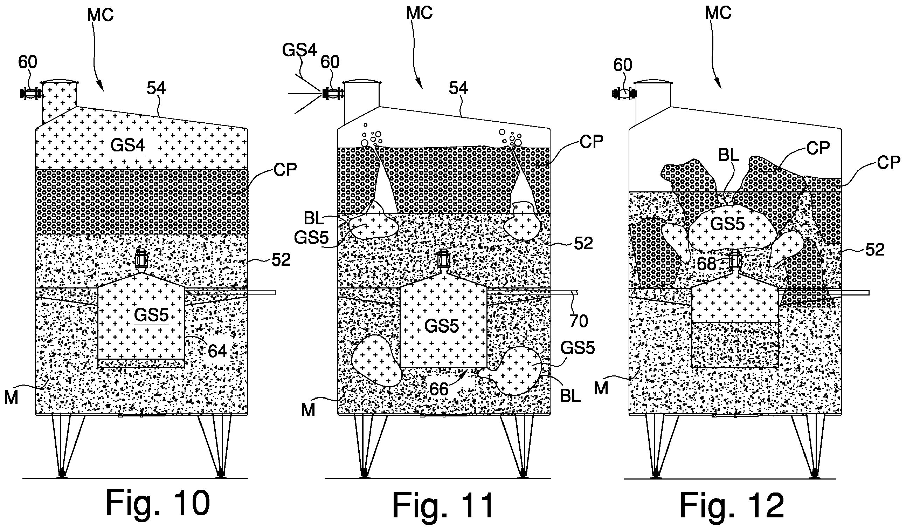

In Fig. 10 the tank 52 filled with must M can be seen. During fermentation a floating marc cap CP forms, and the gas given off by the must M (reference GS4) accumulates both below the ceiling 54 and inside the tank 64 (refer GS5). The mechanism of entrapment of gas GS5 is the same as previously described.

By opening the valve 60 (Fig. 11) the tank 52 is vented and an increase in volume in the gas GS5 is induces, which gas comes out from opening 66 forming large bubbles BL. The bubbles BL rise in the must M to the cap CP and crush it. Through the pipe 70 one can add the gas GS5with appropriate mixtures, e.g. oxygen, to disperse them into the must M.

The ability to break the cap for the fermenter MC is great, guaranteed by the discharge of macro-bubbles BL being impossible using input gas ducts from the outside. The bubbles BL depart from the center of the tank 52 and come out uniformly over 360° from the edge of the opening 66: so they can involve the entire column of must M above and all the cap CP. That would be impossible by a punctiform gas source like an immersed pipe.

The winemaker MC can also be seen as an improved version of EP 0979269, which teaches how to capture gas bubbles generated by fermentation. There a diaphragm is used placed inside the tank, under which a growing gas pocket forms and eventually overflows creating bubbles. So bubbles of larger diameter are formed through the combination with those naturally rising that the diaphragm does not capture.

Although the combined bubbles while rising increase in volume due to the decreasing pressure in the must, the problem remains that the effects are not very effective on the cap. From a winemaking point of view it has been shown that only tumultuous action, e.g. as that obtained in WO 2010 105675, has the desired effect on the cap. This is because the cap is a compact mass of great hardness and thickness (a tramped intertwinement of skins, stems and vegetable fibers), so only a considerable force can tear it apart. Besides, in EP 0979269 the fermentation tank is open at the top, one cannot neither lead fermentation with pressurized must nor vary the pressure in the fermentation tank.

A further improvement in the ability to stir the must and hat is the use of the valve 68 (Fig. 12). Being located in the center of the tank 52 it is able to release effective gas macro-bubbles GS5 just at the center and under the cap CP, unlike EP 0979269 that can create them only peripherally.

The valves 60 and 68 may be controlled independently, e.g. by a PLC, and can therefore be opened simultaneously or at different times, and by durations individually programmable.

## Claims

1. Method of treating a vegetable product in the form of crushed material, comprising
- putting the crushed material (M) into a fermentation tank (12, 40) to let it ferment in pressurized environment and form therein a cap (CP) of solid particles floating on a liquid mass,
- accumulating gaseous products of fermentation (GS2) generated by the pressed material inside a gas accumulation volume (20, 30) and then markedly lowering the pressure within the fermentation tank (12) so as to create a pressure drop within the fermentation tank (12),
the same pressure drop being reflected on the pressure acting on the gaseous products contained in said volume,
so that a pressure differential between the tank and said volume causes
a volume increase for said gaseous products and the spontaneous transfer of the gaseous products in excess from the volume to the liquid mass, so that upon rising they engage the cap;
**characterized by** setting the volume constantly in communication with the liquid mass at a point under the cap.

2. Method according to claim 1, comprising the step of connecting to said tank an external tank (30) through an interruptible fluidic connection (42), so that the accumulation of gaseous products (GS3) can occur by spontaneous migration of the gaseous products in excess into the volume of the second tank.

3. Method according to claim 2, wherein the external tank is arranged above the fermentation tank, or below it using siphoned connections.

4. Method according to any one of the preceding claims, comprising the step of arranging inside the fermentation tank (12) an open cavity (22), so that the accumulation of gaseous products occurs by capturing into the volume of the cavity the fermentation gas bubbles rising naturally in the liquid mass.

5. Method according to claim 4, comprising the step of placing the cavity about at the center of the fermentation tank and from there expelling the gas accumulated in the cavity directly under the cap by opening the cavity.

6. Fermenter for a vegetable product (M) in the form of crushed material, comprising
- a fermentation tank (12, 40, 52) closed or closable to let the crushed material ferment in pressurized environment, which material can form therein a cap (CP) of solid particles floating on a liquid mass,
- a volume (20, 30, 64) adapted to collect gaseous products of fermentation (GS2, GS3, GS5) generated by the crushed material,
- means (14, 48, 60) for markedly lowering the pressure inside the tank so as to create a pressure drop within the fermentation tank (12),
the same pressure drop being reflected on the pressure acting on the gaseous products contained in said volume,
so that the pressure differential between the tank and the volume causes
a volume increase for said gaseous products and the spontaneous transfer of gaseous products in excess from the volume to the liquid mass, so that upon rising they engage the cap;
**characterized in that** the volume is set in constant communication with the liquid mass at a point under the cap.

7. Fermenter according to claim 6, comprising an external tank (30) fluidly connected (42) in interruptible manner with the fermentation tank, so that the accumulation of gaseous products (GS3) can occur by spontaneous migration of the gases in the volume of the external tank.

8. Fermenter according to claim 7, wherein the external tank is arranged above the fermentation tank, or below it with fluid connections comprising a siphon.

9. Fermenter according to any one of the preceding claims 6 to 8, comprising a gas recipient (22) mounted suspended inside the fermentation tank (12) and having an opening facing the bottom of the latter, so that inside the recipient the gaseous products accumulate by capturing the fermentation gas bubbles rising naturally in the liquid mass.

10. Fermenter according to claim 9, comprising vent means (68) mounted on the recipient and adapted to release the gas accumulated therein directly under the cap.

## Patentansprüche

1. Verfahren zur Behandlung eines pflanzlichen Produkts in Form von zerkleinertem Material, umfassend
- Einbringen des zerkleinerten Materials (M) in einen Fermentationstank (12, 40), um es in einer unter Druck stehenden Umgebung gären zu lassen und darin eine Kappe (CP) fester Teilchen zu bilden, die auf einer flüssigen Masse schweben;
- Ansammeln gasförmiger Fermentationsprodukte (GS2), die durch das gepresste Material in einem Gassammelvolumen (20, 30) erzeugt werden, und dann den Druck innerhalb des Fermentationstanks (12) deutlich erniedrigen, um so einen Druckabfall innerhalb des Fermentationstanks (12) zu erzeugen,
der gleiche Druckabfall spiegelt sich in dem Druck wider, der auf die in diesem Volumen enthaltenen gasförmigen Produkte wirkt,
so dass sich eine Druckdifferenz zwischen dem Tank und dem genannten Volumen ergibt
eine Volumenzunahme für die gasförmigen Produkte und die spontane Übertragung der überschüssigen gasförmigen Produkte vom Volumen auf die flüssige Masse, so dass sie beim Aufsteigen mit der Kappe in Eingriff gelangen;
**gekennzeichnet durch** Einstellen des Volumens in ständiger Verbindung mit der flüssigen Masse an einem Punkt unter der Kappe.

2. Verfahren nach Anspruch 1, umfassend den Schritt des Verbindens eines externen Tanks (30) mit dem Tank durch eine unterbrechbare fluidische Verbindung (42), so dass die Ansammlung von gasförmigen Produkten (GS3) durch spontane Migration der gasförmigen Produkte erfolgen kann im Übermaß in das Volumen des zweiten Tanks.

3. Verfahren nach Anspruch 2, **dadurch gekennzeichnet, dass** der Außentank unter Verwendung von Syphonanschlüssen oberhalb des Fermentationstanks oder darunter angeordnet ist.

4. Verfahren nach einem der vorhergehenden Ansprüche, umfassend den Schritt des Anordnens eines offenen Hohlraums (22) innerhalb des Fermentationstanks (12), so dass die Ansammlung von gasförmigen Produkten durch Einfangen des Fermentationsgases in das Volumen des Hohlraums erfolgt Blasen steigen natürlich in der flüssigen Masse auf.

5. Verfahren nach Anspruch 4, umfassend den Schritt des Anordnens des Hohlraums in der Mitte des Fermentationstanks und Ausstoßen des in dem Hohlraum angesammelten Gases direkt unter der Kappe durch Öffnen des Hohlraums.

6. Fermenter für ein pflanzliches Produkt (M) in Form von zerkleinertem Material, umfassend
- einen Fermentationstank (12, 40, 52), der geschlossen oder verschließbar ist, um das zerkleinerte Material in einer unter Druck stehenden Umgebung fermentieren zu lassen, wobei das Material darin eine Kappe (CP) fester Partikel bilden kann, die auf einer flüssigen Masse schweben;
- ein Volumen (20, 30, 64), das zum Sammeln gasförmiger Fermentationsprodukte (GS2, GS3, GS5) geeignet ist, die durch das zerkleinerte Material erzeugt werden,
- Mittel (14, 48, 60) zum deutlichen Absenken des Drucks innerhalb des Tanks, um einen Druckabfall innerhalb des Fermentationstanks (12) zu erzeugen;
der gleiche Druckabfall spiegelt sich in dem Druck wider, der auf die in diesem Volumen enthaltenen gasförmigen Produkte wirkt,
so dass der Druckunterschied zwischen Tank und Volumen entsteht
eine Volumenzunahme für die gasförmigen Produkte und die spontane Übertragung von überschüssigen gasförmigen Produkten aus dem Volumen auf die flüssige Masse, so dass sie beim Aufsteigen mit der Kappe in Eingriff gelangen;
**dadurch gekennzeichnet, dass** das Volumen an einem Punkt unter der Kappe in ständiger Verbindung mit der flüssigen Masse eingestellt wird.

7. Fermenter nach Anspruch 6, der einen äußeren Tank (30) umfasst, der fluidunterbrechbar mit dem Fermentationsbehälter (42) verbunden ist, so dass die Ansammlung von gasförmigen Produkten (GS3) durch spontane Wanderung der Gase im Volumen von erfolgen kann der Außentank.

8. Fermenter nach Anspruch 7, wobei der Außentank oberhalb des Fermentationstanks oder darunter angeordnet ist, wobei Fluidanschlüsse einen Siphon umfassen.

9. Fermenter nach einem der vorangehenden Ansprüche 6 bis 8, umfassend einen Gasbehälter (22), der innerhalb des Fermentationsbehälters (12) hängend angebracht ist und eine Öffnung aufweist, die dem Boden des letzteren zugewandt ist, so dass im Inneren des Empfängers die gasförmigen Produkte angeordnet sind Sammeln Sie durch Auffangen der Gärgasblasen, die natürlich in der flüssigen Masse aufsteigen.

10. Fermenter nach Anspruch 9, umfassend Entlüftungsmittel (68), die an dem Rezipienten angebracht sind und dazu ausgelegt sind, das darin angesammelte Gas direkt unter der Kappe freizusetzen.

## Revendications

1. Procédé de traitement d'un produit végétal sous forme de matériau broyé, comprenant
- placer le matériau broyé (M) dans une cuve de fermentation (12, 40) pour le laisser fermenter dans un environnement sous pression et y former un bouchon (CP) de particules solides flottant sur une masse liquide,
- accumuler les produits de fermentation gazeux (GS2) générés par le matériau pressé à l'intérieur d'un volume d'accumulation de gaz (20, 30), puis baisser nettement la pression dans la cuve de fermentation (12) afin de créer une chute de pression dans la cuve de fermentation (12),
la même chute de pression se répercutant sur la pression agissant sur les produits gazeux contenus dans ledit volume,
de sorte qu'une différence de pression entre le réservoir et ledit volume provoque une augmentation de volume pour lesdits produits gazeux et le transfert spontané des produits gazeux en excès du volume à la masse liquide, de sorte que, lorsqu'ils se lèvent, ils s'engagent dans le bouchon;
**caractérisé par** le réglage constant du volume en communication avec la masse liquide en un point situé sous le bouchon.

2. Procédé selon la revendication 1, comprenant l'étape consistant à connecter au réservoir un réservoir externe (30) par une connexion fluidique interruptible (42), de sorte que l'accumulation de produits gazeux (GS3) puisse se produire par migration spontanée des produits gazeux. en excès dans le volume du deuxième réservoir.

3. Procédé selon la revendication 2, dans lequel le réservoir externe est disposé au-dessus du réservoir de fermentation ou au-dessous de celui-ci en utilisant des connexions siphonnées.

4. Procédé selon l'une quelconque des revendications précédentes, comprenant l'étape consistant à disposer à l'intérieur de la cuve de fermentation (12) une cavité ouverte (22), de sorte que l'accumulation de produits gazeux se produise en capturant dans le volume de la cavité le gaz de fermentation. bulles montantes naturellement dans la masse liquide.

5. Procédé selon la revendication 4, comprenant l'étape consistant à placer la cavité au centre de la cuve de fermentation et à partir de là à expulser le gaz accumulé dans la cavité directement sous le bouchon en ouvrant la cavité.

6. Fermenteur pour produit végétal (M) sous forme de matériau broyé, comprenant
- une cuve de fermentation (12, 40, 52) fermée ou pouvant être fermée pour laisser fermenter le matériau broyé dans un environnement sous pression, lequel matériau peut former dans celui-ci un bouchon (CP) de particules solides flottant sur une masse liquide,
- un volume (20, 30, 64) adapté pour collecter les produits gazeux de fermentation (GS2, GS3, GS5) générés par le matériau broyé,
- des moyens (14, 48, 60) pour abaisser de manière marquée la pression à l'intérieur du réservoir afin de créer une chute de pression dans le réservoir de fermentation (12), la même chute de pression se répercutant sur la pression agissant sur les produits gazeux contenus dans ledit volume,
de sorte que la différence de pression entre le réservoir et le volume provoque
une augmentation de volume pour lesdits produits gazeux et le transfert spontané de produits gazeux en excès du volume à la masse liquide, de sorte que, lorsqu'ils se lèvent, ils s'engagent dans le bouchon;
**caractérisé en ce que** le volume est mis en communication constante avec la masse liquide en un point situé sous le bouchon.

7. Fermenteur selon la revendication 6, comprenant une cuve externe (30) reliée fluidiquement (42) de manière interruptible à la cuve de fermentation, de sorte que l'accumulation de produits gazeux (GS3) peut se produire par migration spontanée des gaz dans le volume de le réservoir externe.

8. Fermenteur selon la revendication 7, dans lequel le réservoir externe est disposé au-dessus du réservoir de fermentation ou au-dessous de celui-ci avec des connexions de fluide comprenant un siphon.

9. Fermenteur selon l'une quelconque des revendications précédentes 6 à 8, comprenant un récipient à gaz (22) monté suspendu à l'intérieur de la cuve de fermentation (12) et présentant une ouverture tournée vers le fond de celle-ci, de manière à ce que les produits gazeux soient à l'intérieur du récipient. s'accumulent en capturant les bulles de gaz de fermentation qui montent naturellement dans la masse liquide.

10. Fermenteur selon la revendication 9, comprenant des moyens d'évent (68) montés sur le récipient et adaptés pour libérer le gaz qui s'y est accumulé directement sous le bouchon.
